(19) Europäisches Patentamt
European Patent Office
Office européen des brevets

(11) Numéro de publication: **0 262 063 B1**

(12) **FASCICULE DE BREVET EUROPEEN**

(45) Date de publication de fascicule du brevet:
**29.01.92**

(51) Int. Cl.5: **C07C 37/86**, C07C 37/88,
C07C 39/32, C07C 39/34,
C07C 39/36

(21) Numéro de dépôt: **87420253.4**

(22) Date de dépôt: **24.09.87**

(54) procédé de stabilisation des mélanges de chloration du phénol et/ou de chlorophénols.

(30) Priorité: **25.09.86 FR 8613557**

(43) Date de publication de la demande:
**30.03.88 Bulletin 88/13**

(45) Mention de la délivrance du brevet:
**29.01.92 Bulletin 92/05**

(84) Etats contractants désignés:
**AT BE CH DE ES FR GB GR IT LI LU NL SE**

(56) Documents cités:
**EP-A- 0 123 233
FR-A- 958 804
US-A- 760 010
US-A- 2 938 059**

(73) Titulaire: **RHONE-POULENC CHIMIE
25, quai Paul Doumer
F-92408 Courbevoie Cédex(FR)**

(72) Inventeur: **Desmurs, Jean
La Jonquière Route de Ternay
Communay F-69360 St-Symphorien
D'Ozon(FR)**
Inventeur: **Ratton, Serge
Hameau de Belmont
Vaulx Milieu F-38090 Villefontaine(FR)**

(74) Mandataire: **Dutruc-Rosset, Marie-Claude et al
RHONE-POULENC CHIMIE Service Brevets
Chimie 25, Ouai Paul Doumer
F-92408 Courbevoie Cédex(FR)**

## Description

La présente invention concerne un procédé de stabilisation des mélanges réactionnels obtenus lors de la chloration du phénol et/ou de chlorophénols en tri-, tétra- et pentachlorophénols.

Lors de la chloration du phénol, des monochlorophénols et des dichlorophénols en trichlorophénols, tétrachlorophénols ou pentachlorophénol, on obtient des mélanges de chloration qui sont colorés et qui évoluent dans le temps. Ainsi on observe notamment, même lors d'un stockage à froid, une augmentation du taux des chlorophénoxyphénols et une évolution de la concentration de certains chlorophénols.

Lorsque de tels mélanges de chloration sont distillés, les produits de la distillation présentent également cette instabilité.

La demanderesse a mis en évidence la présence, dans ces mélanges de chloration, de cétones cycliques insaturées comportant une substitution gem-dichlorée, qui seraient à l'origine de l'évolution de ces mélanges.

La présente invention se propose de résoudre ce problème de l'instabilité des mélanges de chloration.

Plus précisément elle consiste en un procédé de stabilisation d'un mélange de chloration du phénol et/ou de chlorophénols, caractérisé en ce que l'on agite ledit mélange en présence d'au moins un acide protonique fort et/ou un acide de Lewis.

Par acide protonique fort, on entend dans le présente texte un acide protonique ayant une fonction d'acidité Ho inférieure ou égale à - 5.

Comme exemples non limitatifs de tels acides forts, on peut citer l'acide sulfurique, l'acide perchlorique, l'acide trifluorométhanesulfonique, l'acide chlorosulfonique, l'acide fluorosulfonique, l'acide pyrosulfurique ainsi que les résines acides comportant des groupements fluorosulfoniques.

On assimilera également dans ce qui suit aux acides protoniques forts les formes acides des aluminosilicates, les formes acides des argiles et les silices.

Parmi les aluminosilicates acides, on peut citer notamment les zéolithes et les tamis moléculaires et parmi les argiles acides on peut citer notamment les bentonites.

Par acide de Lewis, on entend dans le présent texte, selon la définition usuelle, des composés accepteurs de doublets électroniques.

On peut utiliser notamment les acides de Lewis cités dans l'ouvrage édité par G.A. "Friedel-Crafts and related Reactions", tome I, pages 191 à 197 (1963).

Les acides de Lewis pouvant servir dans le procédé, sont plus particulièrement les halogénures d'éléments des groupes 3a, 4a, 5a, 1b, 2b, 3b, 4b, 5b, 6b, 7b et 8 de la classification périodique des éléments, qui sont liquides ou solides dans les conditions opératoires, tels que les chlorures, bromures, fluorures et iodures d'aluminium, de gallium, d'étain, de phosphore, d'antimoine, d'arsenic, de bismuth, de titane, de tantale, de tellure, de sélénium, de zirconium, de hafnium, de vanadium, de samarium, de nobium, de tungstène, de platine, de molybdène, de fer, de cobalt, de nickel, de zinc et de cadmium.

Comme exemples spécifiques de tels halogénures, on peut citer le chlorure d'aluminium, le bromure d'aluminium, le chlorure ferrique, le bromure ferrique, le tétrachlorure de titane, le tétrachlorure de zirconium, le chlorure de platine, le trichlorure de vanadium, le chlorure de samarium, le chlorure de tellure, le chlorure de sélénium, le pentafluorure d'antimoine, le trichlorure de bismuth, le chlorure stannique, le trichlorure de gallium, le tétrachlorure de hafnium, le tribromure de phosphore.

Certains acides de Lewis, les chlorures des éléments indiqués ci-avant, peuvent être préparés in situ par introduction dudit élément dans le mélange de chloration.

Les cétones cycliques insaturées gem-dichlorées sont essentiellement des cyclohexadiénones gem-dichlorées comportant par ailleurs 1, 2, 3 ou 4 autres atomes de chlore sur différents atomes de carbone du cycle et des cyclohexénones gem-dichlorées comportant également par ailleurs 1 à 6 autres atomes de chlore sur différents atomes de carbone du cycle.

Ce sont d'une part les dichloro-4,4-cyclohexadiène-2,5 ones et les dichloro-6,6 cyclohexadiène-2,4 ones comportant en outre 1 à 4 atomes de chlore.

A titre d'exemples des principales cyclohexadiènones gem-dichlorées, on peut citer :
la dichloro-6,6 cyclohexadiène-2,4 one,
la dichloro-4,4 cyclohexadiène-2,5 one,
la tétrachloro-2,4,4,6 cyclohexadiène-2,5 one,
la tétrachloro-2,4,6,6 cyclohexadiène-2,4 one,
la pentachloro-2,3,4,4,6 cyclohexadiène-2,5 one,
la pentachloro-2,4,5,6,6 cyclohexadiène-2,4 one,
la pentachloro-2,3,4,6,6 cyclohexadiène-2,4 one,
l'hexachloro-2,3,4,4,5,6 cyclohexadiène-2,5 one,

et l'hexachloro-2,3,4,5,6,6 cyclohexadiène,2,4 one.

Ce sont d'autre part les dichloro-2,2 cyclohexène-3 ones, les dichloro-6,6 cyclohexène-2 ones, les dichloro-4,4 cyclohexène-2 ones, les dichloro-6,6 cyclohexène-3 ones comportant en outre 1 à 6 atomes de chlore.

A titre d'exemples des principales cyclohexènones gem-dichlorées, on peut citer :
la pentachloro-2,4,5,6,6 cyclohexène-2 one,
l'hexachloro-2,4,4,5,6,6 cyclohexène-2 one,
l'hexachloro-2,2,4,5,6,6 cyclohexène-3 one,
l'heptachloro-2,4,4,5,5,6,6 cyclohexène-2 one,
l'heptachloro-2,2,3,4,5,6,6 cyclohexène-3 one,
l'heptachloro-2,3,4,4,5,5,6 cyclohexène-2 one,
l'heptachloro-2,3,4,4,5,6,6 cyclohexène-2 one,
l'octachloro-2,3,4,4,5,5,6,6 cyclohexène-2 one,
et l'octachloro-2,2,3,4,5,5,6,6 cyclohexène-3 one.

La température à laquelle sont agités le mélange issu de la chloration du phénol et/ou du chlorophénols et le ou les acides forts et/ou acides de Lewis varie dans de larges limites, par exemple entre 20° C et 200° C.

De préférence cependant, pour avoir une bonne réaction, la température sera de 40° C à 180° C et le plus souvent de 60° C à 150° C.

La durée de traitement est très variable en fonction de la température, de la quantité de cétones cycliques gem-dichlorées contenues dans la mélange et l'acide fort ou de l'acide de Lewis utilisé. Elle peut varier par exemple de quelques minutes à plusieurs dizaines d'heures.

En général, elle se situe entre 1 heure et 15 heures, sans que ces nombres aient une valeur critique.

La quantité d'acide fort et/ou d'acide de Lewis dépend bien évidemment de la teneur en cétones cycliques gem-dichlorées du mélange et de leur nature. Ces cétones sont généralement dosées dans le mélange par chromatographie en phase liquide à double détection : un détecteur à ultra-violets pour l'ensemble des composés du mélange et un détecteur électrochimique spécifiquement pour les cétones cycliques gem-dichlorées ou par un dosage global en électrochimie.

Pour avoir une bonne stabilisation des mélanges de chloration, il faut introduire une quantité d'acide fort et/ou d'acide de Lewis au moins égale à 0,05 mole pour 1 mole de cétones cycliques gem-dichlorées.

Comme il n'est pas toujours aisé de déterminer avec précision la nature des différentes cétones cycliques insaturées gem-dichlorées des mélange de chloration, il est préférable d'introduire une quantité d'acide fort et/ou d'acide de Lewis de 0,2 à 10 moles par mole de cétones cycliques insaturées gem-dichlorées.

Les exemples qui suivent illustrent la présente invention.

EXEMPLES 1 à 16

Dans un réacteur en verre de 10 cm$^3$ avec agitation, on charge :
- trichloro-2,4,6 phénol :        1,97 g (0,010 mole)
- tétrachloro-2,4,4,6 cyclohexadiène-2,5 one :        0,23 g (0,001 mole)
- composé acide :        quantité indiqué dans le tableau (I).

On chauffe le mélange sous agitation pendant 8 heures (sauf pour l'exemple 16 dans lequel la durée est de 24 heures) à 70° C (sauf pour l'exemple 15 dans lequel la température est de 125° C).

On analyse la masse réactionnelle par chromatographie en phase liquide, à l'aide d'une double détection UV (totalité des composés chlorés) et ampérométrie (cétones cycliques insaturées gem-dichlorées).

On effectue dans les mêmes conditions un essai témoin avec les mêmes charges à l'exception du composé acide.

Le tableau (I) ci-après indique la nature et la quantité de composé acide utilisé ainsi que les résultats des analyses effectuées après le traitement.
TT % = taux de transformation.
RT % = rendement par rapport à la chlorocyclohexadiènone transformée.

**Tableau I**

| ESSAIS | Composé acide quantité utilisée | TT % de la chlorocyclohexadiènone | RT en tri-chloro-2,4,6 phénol | RT % en tétrachloro-2,3,4,6 phénol | RT en pentachloro-phénol | chlorophé-noxyphénols (en mmol) |
|---|---|---|---|---|---|---|
| Témoin A | Néant | 2 | 0 | 0 | 0 | 0 |
| Exemple 1 | $CF_3SO_3H$ : 2,5 mmol | 100 | 0 | 78,4 | 0 | 0 |
| Exemple 2 | $CF_3SO_3H$ : 0,48 mmol | 100 | 0 | 78,4 | 0 | 0 |
| Exemple 3 | $CF_3SO_3H$ : 0,13 mmol | 100 | 0 | 72,3 | 0 | 0 |
| Exemple 4 | $AlCl_3$ : 2,5 mmol | 100 | 0 | 78,7 | 0 | 0,325 |
| Exemple 5 | $AlCl_3$ : 0,031 mmol | 100 | 0 | 64,9 | 0 | 0 |
| Exemple 6 | $AlCl_3$ : 0,31 mmol | 100 | 0 | 71,3 | 0 | 0 |

.../...

Tableau I - (suite)

| ESSAIS | Composé acide quantité utilisée | TT % de la chlorocyclo-hexadiènone | RT en tri-chloro-2,4,6 phénol | RT % en tétrachloro-2,3,4,6 phénol | RT en pentachloro-phénol | chlorophé-noxyphénols (en mmol) |
|--------|---------------------------------|-----------------------------------|-------------------------------|-----------------------------------|--------------------------|-------------------------------|
| Exemple 7 | ZrCl4 : 2,5 mmol | 100 | 0 | 100 | 0 | 0 |
| Exemple 8 | SiO2 : 2,5 mmol | 33 | 0 | 23 | 0 | 0 |
| Exemple 9 | bentonite : 0,25 g séchée 6 h à 200°C | 66 | 0 | 17 | 0 | 0 |
| Exemple 10 | Te : 0,50 mmol | 100 | 0 | 94 | 0 | 0,37 |
| Exemple 11 | ZrCl4 : 2,5 mmol | 100 | 0 | 100 | 0 | 0,37 |
| Exemple 12 | Se : 2,5 mmol | 100 | 0 | 36 | 0 | 0,37 |
| Exemple 13 | PtCl4 : 2,5 mmol | 100 | 0 | 89 | 0 | 0,24 |

.../...

Tableau I - (suite)

| ESSAIS | Composé acide quantité utilisée | TT % de la chlorocyclo-hexadiènone | RT en tri-chloro-2,4,6 phénol | RT % en tétrachloro-2,3,4,6 phénol | RT en pentachloro-phénol | chlorophé-noxyphénols (en mmol) |
|---|---|---|---|---|---|---|
| Exemple 14 | VCl3 : 2,5 mmol | 74,3 | 0 | 16 | 0 | 0 |
| Exemple 15 | SmCl3, 6 H2O : 2,5 mmol | 93 | 0 | 36,4 | 0 | 0 |
| Exemple 16 | SiO2 : 2,5 mmol | 73 | 0 | 57 | 0 | 0,06 |

EXEMPLES 17 à 22

Dans le réacteur décrit dans les exemples 1 à 16, on charge :
- pentachloro-2,3,4,4,6 cyclohexadiène-2,5 one : 0,265 g (0,001 mole)

6

- trichloro-2,4,6 phénol (ou tétrachloro-2,3,4,6 phénol) : 0,010 mole
- composé acide : 0,0025 mole.

On opère comme indiqué dans les exemples 1 à 16.

Durée des essais : 8 heures à 70° C.

Le tableau (II) ci-après indique la nature du composé phénolique et du composé acide utilisés, ainsi que les résultats des analyses effectuées après le traitement.

Tableau II

| ESSAIS | Chlorophénol Composé acide | TT % de la chlorocyclo-hexadiènone | RT % en tétra-chloro-2,3,4,6 phénol | RT % en penta-chlorophénol | chlorophénoxy-phénols en mmol |
|---|---|---|---|---|---|
| Témoin B | trichloro-2,4,6 phénol néant | 27 | 11,2 | 0 | 0 |
| Témoin C | tétrachloro2,3,4,6 phénol néant | 7 | 0 | 0 | 0 |
| Exemple 17 | trichloro-2,4,6 phénol AlCl3 | 100 | 88,0 | 0 | 0,17 |
| Exemple 18 | trichloro-2,4,6 phénol FeCl3 | 100 | 50 | 0 | 0,17 |
| Exemple 19 | trichloro-2,4,6 phénol TiCl4 | 95,9 | 29 | 58,9 | 0 |
| Exemple 20 | trichloro-2,4,6 phénol CF3SO3H | 100 | 10 | 67,9 | 0 |
| Exemple 21 | trichloro-2,4,6 phénol H2SO4 | 70,7 | 42,9 | 29 | 0 |
| Exemple 22 | tétrachloro-2,3,4,6 phénol AlCl3 | 100 | 0 | 74,3 | 0 |

EXEMPLES 23 à 26

Dans un réacteur en verre de 10 cm$^3$, avec agitation on charge :
- trichloro-2,4,6 phénol ou tétrachloro-2,3,4,6 phénol : 0,010 mole
- hexachloro-2,3,4,4,5,6 cyclohexadiène-2,5 one : 0,30 g (0,001 mole)
- composé acide : 0,0025 mole.

On opère comme dans les exemples 1 à 16 pendant 8 heures à 70°C.

Le tableau III ci-après indique la nature du composé phénolique et du composé acide utilisés, ainsi que les résultats des analyses effectuées après le traitement.

**Tableau III**

| ESSAIS | Chlorophénol Composé acide | TT % de la chlorocyclohexadiènone | RT % en pentachlorophénol | chlorophénoxy-phénols en mmol |
|---|---|---|---|---|
| Témoin D | trichloro-2,4,6 phénol néant | 2 | 2 | 0 |
| Exemple 23 | trichloro-2,4,6 phénol AlCl3 | 100 | 100 | 0 |
| Exemple 24 | trichloro-2,4,6 phénol CF3SO3H | 100 | 99 | 0 |
| Témoin E | tétrachloro-2,3,4,6 phénol néant | 2 | 2 | 0 |
| Exemple 25 | tétrachloro-2,3,4,6 phénol AlCl3 | 100 | 100 | 0 |
| Exemple 26 | tétrachloro-2,3,4,6 phénol CF3SO3H | 100 | 100 | 0 |

EXEMPLES 27 à 38

Dans un réacteur en verre de 30 cm$^3$ placé dans un four à 70°C et comportant une agitation magnétique, on charge :

- tétrachloro-2,4,4,6 cyclohexadiène-2,5 one-l : 0,70 g
- trichloro-2,4,6 phénol : 5,93 g
- composé acide : 0,10 g

On fait fondre la masse réactionnelle avant de placer le réacteur dans le four. On suit l'évolution de la réaction par des prélèvements.

Le tableau IV ci-après indique la nature du composé acide utilisé, ainsi que les résultats des analyses effectuées après le traitement.

Tableau IV

| ESSAIS | Composé acide | Durée | TT % de la chlorocyclo- hexadiènone | RT % en tri- chloro-2,4,6 phénol | RT % en tétra- chloro phénol | chlorophénoxy- phénols % en poids |
|---|---|---|---|---|---|---|
| Témoin A | néant | 8 h | 2 | 0 | 0 | 0 |
| Exemple 27 | ZrCl4 | 30 min | 100 | 0 | 78,3 | 2,2 |
| Exemple 28 | TiCl4 | 60 min | 30,4 | 0 | 35,0 | 1,4 |
| Exemple 29 | HfCl4 | 15 min | 100 | 0 | 80,3 | 0 |
| Exemple 30 | ClSO3H | 30 min | 100 | 0 | 70,0 | 0 |
| Exemple 31 | FeCl3 | 30 min | 100 | 0 | 71,8 | 3,0 |
| Exemple 32 | FeBr3 | 30 min | 100 | 0 | 59,4 | 1,2 |

.../...

EP 0 262 063 B1

Tableau IV (suite)

| ESSAIS | Composé acide | Durée | TT % de la chlorocyclo-hexadiènone | RT % en tri-chloro-2,4,6 phénol | RT % en tétra-chloro phénol | chlorophénoxy-phénols % en poids |
|---|---|---|---|---|---|---|
| Exemple 33 | GaCl3 | 15 min | 100 | 0 | 80,1 | 3,0 |
| Exemple 34 | AlCl3 | 15 min | 100 | 0 | 68,7 | 10,0 |
| Exemple 35 | AlBr3 | 15 min | 100 | 0 | 15,8 | 13,0 |
| Exemple 36 | PBr3 | 30 min | 46,6 | 0 | 35,4 | 0 |
| Exemple 37 | TeCl4 | 60 min | 100 | 0 | 72,5 | 1,0 |
| Exemple 38 | SbCl3 | 35 min | 100 | 0 | 84,1 | 5,0 |

Revendications

1. Procédé de stabilisation d'un mélange issu de la chloration du phénol et/ou de chlorophénols, caractérisé en ce que l'on agite ledit mélange en présence d'au moins un acide protonique fort et/ou un acide de Lewis.

2. Procédé selon la revendication 1, caractérisé en ce que l'acide protonique a une fonction d'acidité Ho inférieure ou égale à - 5.

3. Procédé selon la revendication 2, caractérisé en ce que l'acide protonique fort est choisi parmi l'acide sulfurique, l'acide perchlorique, l'acide trifluorométhanesulfonique, l'acide chlorosulfonique, l'acide fluorosulfonique, l'acide pyrosulfurique ainsi que les résines acides comportant des groupements fluorosulfoniques.

4. Procédé selon la revendication 1, caractérisé en ce que l'acide fort est choisi parmi les formes acides des aluminosilicates, les formes acides des argiles et les silices.

5. Procédé selon la revendication 4, caractérisé en ce que l'acide fort est choisi parmi les zéolithes, les tamis moléculaires, les bentonites et les silices.

6. Procédé selon la revendication 1, caractérisé en ce que l'acide de Lewis est choisi parmi les halogénures d'éléments des groupes 3a, 4a, 5a, 1b, 2b, 3b, 4b, 5b,6b, 7b et 8 de la classification périodique des éléments qui sont liquides ou solides dans les conditions opératoires.

7. Procédé selon l'une des revendications 1 ou 6, caractérisé en ce que l'acide de Lewis est choisi parmi les chlorures, bromures, fluorures et iodures d'aluminium, de gallium, d'étain, de phosphore, d'antimoine, d'arsenic, de bismuth, de titane, de tantale, de tellure, de sélénium, de zirconium, de hafnium, de vanadium, de samarium, de nobium, de tungstène, de platine, de molybdène, de fer, de cobalt, de nickel, de zinc et de cadmium.

8. Procédé selon l'une des revendications 1, 6 ou 7, caractérisé en ce que l'acide de Lewis est choisi parmi le chlorure d'aluminium, le bromure d'aluminium, le chlorure ferrique, le bromure ferrique, le tétrachlorure de titane, le tétrachlorure de zirconium, le chlorure de platine, le trichlorure de vanadium, le chlorure de samarium, le chlorure de tellure, le chlorure de sélénium, le pentafluorure d'antimoine, le trichlorure de bismuth, le chlorure stannique, le trichlorure de gallium, le tétrachlorure de hafnium, le tribromure de phosphore.

9. Procédé selon l'une des revendications 7 ou 8, caractérisé en ce que l'acide de Lewis est préparé in situ par introduction dans le mélange de chloration du métal correspondant.

10. Procédé selon l'une des revendications 1 à 9, caractérisé en ce que l'on opère à une température de 20°C à 200°C et de préférence de 40°C à 180°C.

11. Procédé selon l'une des revendications 1 à 10, caractérisé en ce que l'on opère à une température de 60°C à 150°C.

12. Procédé selon l'une des revendications 1 à 11, caractérisé en ce que le rapport molaire acide protonique et/ou acide de Lewis/cétones cycliques gem-dichlorées présentes dans le mélange issu de la chloration du phénol et/ou de chlorophénols est au moins égal à 0,05 et de préférence de 0,2 à 5.

**Claims**

1. Process for stabilizing a mixture that is produced in the chlorination of phenol and/or chloro-phenols, characterised in that the said mixture is stirred in the presence of at least one strong protonic acid and/or a Lewis acid.

2. Process according to claim 1, characterised in that the protonic acid has an acidity function Ho of less than or equal to -5.

3. Process according to claim 2, characterised in that the strong protonic acid is chosen from sulphuric acid, perchloric acid, trifluoromethanesulphonic acid, chlorosulphonic acid, fluorosulphonic acid, pyrosulphuric acid, and acidic resins containing fluorosulphonic groups.

4. Process according to claim 1, characterised in that the strong acid is chosen from the acid forms of aluminosilicates, the acid forms of clays, and silicas.

5. Process according to claim 4, characterised in that the strong acid is chosen from zeolites, molecular sieves, bentonites and silicas.

6. Process according to claim 1, characterised in that the Lewis acid is chosen from the halides of elements of Groups 3a, 4a, 5a, 1b, 2b, 3b, 4b, 5b, 6b, 7b and 8 of the Periodic Classification of the elements which are liquid or solid under the working conditions.

7. Process according to either of claims 1 and 6, characterised in that the Lewis acid is chosen from the chlorides, bromides, fluorides and iodides of aluminium, gallium, tin, phosphorus, antimony, arsenic, bismuth, titanium, tantalum, tellurium, selenium, zirconium, hafnium, vanadium, samarium, niobium, tungsten, platinum, molybdenum, iron, cobalt, nickel, zinc and cadmium.

8. Process according to one of claims l, 6 and 7, characterised in that the Lewis acid is chosen from aluminium chloride, aluminium bromide, ferric chloride, ferric bromide, titanium tetrachloride, zirconium tetrachloride, platinum chloride, vanadium trichloride, samarium chloride, tellurium chloride, selenium chloride, antimony pentafluoride, bismuth trichloride, stannic chloride, gallium trichloride, hafnium tetrachloride and phosphorus tribromide.

9. Process according to either of claims 7 and 8, characterised in that the Lewis acid is prepared in situ by introducing the corresponding metal into the chlorination mixture.

10. Process according to one of claims 1 to 9, characterised in that the reaction is performed at a temperature of $20\degree C$ to $200\degree C$, and preferably from $40\degree C$ to $180\degree C$.

11. Process according to one of claims 1 to 10, characterised in that the reaction is performed at a temperature of $60\degree C$ to $150\degree C$.

12. Process according to one of claims 1 to 11, characterised in that the mole ratio of protonic acid and/or Lewis acid to gem-dichlorinated cyclic ketones present in the mixture that is produced in the chlorination of phenol and/or chlorophenols is at least equal to 0.05 and is preferably from 0.2 to 5.

**Patentansprüche**

1. Verfahren zur Stabilisierung eines aus der Chlorierung von Phenol und/oder von Chlorphenolen entstandenen Gemischs, dadurch gekennzeichnet, daß man dieses Gemisch in Gegenwart von wenigstens einer starken Protonensäure und/oder einer Lewis-Säure bewegt.

2. Verfahren nach Anspruch 1, dadurch gekennzeichnet, daß die Protonensäure eine Säurefunktion $H_o$ von kleiner oder gleich -5 hat.

3. Verfahren nach Anspruch 2, dadurch gekennzeichnet, daß die starke Protonensäure aus Schwefel-, Perchlor-, Trifluormethansulfon-, Chlorsulfon-, Fluorsulfon-, Dischwefelsäure sowie Fluorsulfonsäuregruppen enthaltenden Säureharzen ausgewählt ist.

4. Verfahren nach Anspruch 1, dadurch gekennzeichnet, daß die starke Säure aus den sauren Formen von Aluminosilicaten, den sauren Formen von Tonen und aus Siliciumoxiden ausgewählt ist.

5. Verfahren nach Anspruch 4, dadurch gekennzeichnet, daß die starke Säure aus Zeolithen, Molekularsieben, Bentoniten und Siliciumoxiden ausgewählt ist.

6. Verfahren nach Anspruch 1, dadurch gekennzeichnet, daß die Lewis-Säure aus den Halogeniden der Elemente der Gruppen 3a, 4a, 5a, 1b, 2b, 3b, 4b, 5b, 6b, 7b und 8 des Periodensystems, die unter Arbeitsbedingungen flüssig oder fest sind, ausgewählt ist.

7. Verfahren nach einem der Ansprüche 1 oder 6, dadurch gekennzeichnet, daß die Lewis-Säure

14

ausgewählt ist aus den Chloriden, Bromiden, Fluoriden und Jodiden von Aluminium, Gallium, Zinn, Phosphor, Antimon, Arsen, Wismut, Titan, Tantal, Tellur, Selen, Zirkonium, Hafnium, Vanadium, Samarium, Niob, Wolfram, Platin, Molybdän, Eisen, Cobalt, Nickel, Zink und Cadmium.

8.  Verfahren nach einem der Ansprüche 1, 6 oder 7, dadurch gekennzeichnet, daß die Lewis-Säure ausgewählt ist aus Aluminiumchlorid, Aluminiumbromid, Eisenchlorid, Eisenbromid, Titantetrachlorid, Zirkoniumtetrachlorid, Platinchlorid, Vanadiumtrichlorid, Samariumchlorid, Tellurchlorid, Selenchlorid, Antimonpentafluorid, Wismuttrichlorid, Zinnchlorid, Galliumtrichlorid, Hafniumtetrachlorid, Phosphortribromid.

9.  Verfahren nach einem der Ansprüche 7 oder 8, dadurch gekennzeichnet, daß die Lewis-Säure in situ durch Hinzufügen des entsprechenden Metalls zum Chlorierungsgemisch hergestellt ist.

10. Verfahren nach einem der Ansprüche 1 bis 9, dadurch gekennzeichnet, daß man bei einer Temperatur zwischen 20 und 200 $^{\circ}$C und vorzugsweise zwischen 40 und 180 $^{\circ}$C arbeitet.

11. Verfahren nach einem der Ansprüche 1 bis 10, dadurch gekennzeichnet, daß man bei einer Temperatur zwischen 60 und 150 $^{\circ}$C arbeitet.

12. Verfahren nach einem der Ansprüche 1 bis 11, dadurch gekennzeichnet, daß das Molverhältnis der Protonensäure und/oder Lewis-Säure zu den gem-dichlorierten cyclischen Ketonen, die in dem aus der Chlorierung von Phenol und/oder Chlorphenolen entstandenen Gemisch vorhanden sind, wenigstens gleich 0,05 und vorzugsweise 0,2 bis 5 ist.